# FASCICULE DE BREVET EUROPEEN

(11) **EP 2 661 251 B1**
(45) Date de publication et mention de la délivrance du brevet: **11.07.2018**
(21) Numéro de dépôt: 12700029.7
(22) Date de dépôt: 06.01.2012
(51) Int. Cl.: A61K 9/00, A61K 9/10, A61K 31/573, A61K 31/58, A61K 47/26, A61K 47/38

(54) **PROCÉDÉ DE PRÉPARATION DE SUSPENSIONS AQUEUSES PHARMACEUTIQUES COMPRENANT UN MÉDICAMENT EFFICACE DANS LE TRAITEMENT DES RHINITES**
VERFAHREN ZUR HERSTELLUNG VON WÄSSRIGEN PHARMAZEUTISCHEN SUSPENSIONEN MIT EINEM ARZNEIMITTEL ZUR WIRKSAMEM BEHANDLUNG VON RHINITIS
METHOD FOR PREPARING AQUEOUS PHARMACEUTICAL SUSPENSIONS INCLUDING A DRUG THAT IS EFFECTIVE IN TREATING RHINITIS

(30) Priorité: 06.01.2011 FR 1150104
(43) Date de publication de la demande: 13.11.2013
(73) Titulaire: Substipharm Developpement, 75016 Paris (FR)
(72) Inventeur: ANDRIEUX, Florence, F-92190 Meudon (FR); TERRASSIN, Laurent, F-33700 Merignac (FR)
(74) Mandataire: Regimbeau
(86) Numéro de dépôt international: PCT/EP2012/050189
(87) Numéro de publication internationale: WO 2012/093171

(56) Documents cités:
- WO-A1-97/46243
- WO-A2-01/26658
- US-A- 6 127 353
- US-A1- 2003 049 322

## Description

L'invention comme revendiquée, a pour objet un procédé de préparation de suspensions aqueuses pharmaceutiques comprenant un médicament efficace dans le traitement des rhinites.

Par le traitement des rhinites, on vise plus particulièrement le traitement des diverses formes de rhinite, en particulier des rhinites allergiques. Les rhinites sont des inflammations, hypersécrétions et/ou écoulements, d'origine virale ou allergique, qui affectent les muqueuses du nez et des voies respiratoires supérieures, en particulier celles de la cavité nasale et de la partie nasale du pharynx.

Les rhinites sont le plus souvent traitées à l'aide d'agents anti-inflammatoires stéroïdiens, tels que l'acétonide de triamcinolone, administrés par pulvérisation nasale. Une telle pulvérisation nasale permet un dépôt de l'agent anti-inflammatoire stéroïdien au niveau des muqueuses des parois nasales et cet agent exerce son action pharmacologique pendant qu'il est en contact avec les tissus corporels, en particulier lorsqu'il est en contact avec les muqueuses nasales.

Pour une efficacité maximale, une composition pharmaceutique contenant un tel agent doit être formulée de manière à pouvoir assurer une administration du médicament au niveau de l'ensemble des muqueuses des cavités nasales (les tissus cibles) où il exerce son action pharmacologique. En outre, le médicament doit rester en contact avec les tissus cibles pendant une période suffisamment longue pour assurer un traitement efficace. Afin de rester en contact avec les tissus cibles, la composition pharmaceutique doit présenter une viscosité élevée. En parallèle, la composition doit présenter, au moment de sa pulvérisation, une viscosité suffisamment faible pour pouvoir être pulvérisée. Ainsi, la composition pharmaceutique doit présenter les propriétés rhéologiques suivantes : viscosité élevée au repos, viscosité faible sous agitation (composition ayant un comportement rhéofluidifiant).

La composition pharmaceutique doit présenter des propriétés satisfaisantes de stabilité et de durée de conservation, et elle ne doit pas inclure de constituants qui sont considérés comme nuisibles à l'environnement, par exemple, destructeurs de l'ozone. La composition ne comprend donc pas d'agent de propulsion (tel que les chlorofluorocarbures, les hydrofluorocarbures et les perfluorocarbures).

Le brevet européen n° 938 345 décrit une composition nasale répondant à ces différents objectifs. La formulation développée comprend
- 0,055 % en poids d'acétonide de triamcinolone micronisée (taille de particules comprise entre 1 et 20 microns)
- 2,0 % en poids d'un mélange de cellulose microcristalline et de carboxyméthylcellulose (agent de suspension qui contribue aux propriétés thixotropes de la formulation)
- 0,004 % en poids de polysorbate 80 (agent dispersant)
- 0,05 % en poids d'ethylène diamine tétraacétate disodique - EDTA-(agent anti-microbien et chélatant)
- 0,03 % en poids de chlorure de benzalkonium, solution à 50% (agent anti-microbien)
- 5,0 % en poids de dextrose (agent iso-osmotique)
- 92,96 % en poids d'eau

La formulation est préparée par un procédé comprenant les étapes suivantes :
A) formation d'une solution aqueuse de l'agent dispersant (polysorbate 80) et combinaison de cette solution avec les particules solides de principe actif (acétonide de triamcinolone micronisé) pour former une dispersion de particules. A cette dispersion peut être ajouté l'agent anti-microbien ;
B) ajout de l'agent de suspension (mélange de cellulose microcristalline et de carboxyméthylcellulose) à une solution aqueuse acide pour former une suspension thixotrope. A cette dispersion peut être ajouté l'agent iso-osmotique et l'agent chélatant ; et
C) combinaison des deux suspensions en introduisant une des suspensions (celle qui comprend les particules solides de principe actif) par le bas dans l'autre suspension.

Un tel procédé nécessite deux cuves distinctes pour préparer les deux suspensions. En outre, un tel procédé nécessite pour la préparation de la suspension thixotrope d'une part un dispositif de mélange particulier, à savoir un mélangeur oscillant à vitesse variable (onéreux), et d'autre part des conditions de mélanges douces. Ainsi, à la lecture de l'exemple 1 du brevet européen n° 938 345, on constate que l'étape B) nécessite plusieurs dispositifs (un mélangeur oscillant à vitesse variable, un agitateur à vitesse variable et un disperseur à vitesse fixe) et comprend plusieurs phases d'arrêts et de redémarrage desdits dispositifs. Ces précautions sont prises pour ne pas casser la suspension formée par l'agent de suspension afin d'en conserver les propriétés thixotropes.

Au contraire, les inventeurs ont constaté qu'il n'était pas nécessaire de respecter ces conditions douces de mélange et qu'un procédé plus économique mais plus violent pouvait être utilisé. La suspension aqueuse obtenue par ce procédé présente des propriétés rhéologiques appropriées à son utilisation finale ; à savoir une viscosité élevée au repos, mais une viscosité faible sous agitation.

L'invention comme revendiquée a donc pour objet un procédé industriel de préparation d'une suspension aqueuse d'un agent anti-inflammatoire stéroïdien comprenant un agent dispersant, un agent de suspension et un ou plusieurs excipients choisis dans le groupe constitué par des agents antimicrobiens, des agents chélatants, des agents iso-osmotiques, des agents antioxydants, et leurs mélanges, caractérisé en ce qu'il comprend les étapes successives suivantes :
a) préparation dans un réacteur (1) d'une dispersion comprenant
   - de l'eau,
   - tous lesdits un ou plusieurs excipients ;
   - l'agent dispersant qui est un agent tensio-actif hydrophile et non ionique, et
   - l'agent anti-inflammatoire stéroïdien sous forme de particules ;
b) ajout, à la dispersion obtenue suite à l'étape a), de l'agent de suspension tel quel dans ce même réacteur (1) ;
c) le cas échéant dilution, par ajout d'eau dans ce même réacteur (1), et/ou ajustement du pH de ladite suspension obtenue suite à l'étape b), et
d) récupération de la suspension aqueuse.

L'étape a) comprend avantageusement trois étapes successives distinctes :
i) dissolution dans l'eau de l'agent dispersant et desdits un ou plusieurs excipients ; puis
ii) ajout de l'agent anti-inflammatoire stéroïdien, sous forme de particules, à la solution obtenue suite à l'étape i) et formation de la dispersion ; et
iii) homogénéisation de la dispersion formée à l'étape ii).

Le procédé selon l'invention se caractérise donc en ce qu'un seul réacteur est mis en oeuvre. Contrairement au procédé de l'art antérieur, l'agent de suspension n'est pas préalablement dissout dans une solution aqueuse acide. Ainsi, contrairement au procédé de l'art antérieur, les solutions de dispersion et de suspension ne sont pas préparées dans deux réacteurs différents.

L'eau est présente dans la suspension en une quantité importante. La teneur en eau dans la suspension est avantageusement supérieure à 85 % en poids, par rapport au poids total de la suspension, plus avantageusement d'au moins 90 % en poids, par rapport au poids total de la suspension aqueuse. La majorité de l'eau, avantageusement plus de 70 % en poids par rapport au poids total de l'eau, est introduite dès l'étape a). La dispersion de l'étape a) comprend donc avantageusement au moins 85 % en poids d'eau, par rapport au poids total de la dispersion, plus avantageusement au moins 90 % en poids d'eau, par rapport au poids total de la dispersion.

L'agent anti-inflammatoire stéroïdien est avantageusement choisi dans le groupe constitué par l'acétonide de triamcinolone et le mométasone furoate monohydraté. L'agent anti-inflammatoire stéroïdien est avantageusement l'acétonide de triamcinolone. Cet agent anti-inflammatoire stéroïdien est également désigné dans la description par l'emploi de termes tels que médicament, principe actif, actif.

L'acétonide de triamcinolone est une substance hydrophobe, qui peut être mise en dispersion dans une solution aqueuse en utilisant le procédé selon l'invention. L'acétonide de triamcinolone est utilisée sous la forme de particules, lesdites particules ont une taille telle que le principe actif peut facilement être dispersé. On utilisera de préférence de l'acétonide de triamcinolone micronisée. Les particules de principe actif auront alors avantageusement une taille telle que 99,0 % en poids des particules passent au travers d'un tamis de 10 µm, et 70,0 % en poids des particules passent au travers d'un tamis de 5 µm.

Le médicament est présent dans la suspension en une concentration pharmaceutiquement efficace. Une telle concentration changera selon la condition à traiter et la nature de l'individu traité. Généralement, la teneur en agent anti-inflammatoire stéroïdien dans la suspension est comprise entre 0,001 % et 2 % poids, de préférence entre 0,01 % et 0,2 % en poids, par rapport au poids total de la suspension. De préférence la suspension contient de 0,055% en poids d'agent anti-inflammatoire stéroïdien, par rapport au poids total de la suspension. Ici et dans ce qui suit, sauf indications contraires, les teneurs en chacun des composés (principe actif et excipients) sont données en poids, par rapport au poids total de la suspension aqueuse, qui est la suspension obtenue à la fin du procédé objet de l'invention.

Ces particules d'agent anti-inflammatoire stéroïdien sont uniformément dispersées dans la composition aqueuse en utilisant notamment un agent dispersant pharmaceutiquement acceptable, qui, en mouillant les particules de principe actif, facilite leur dispersion dans la phase aqueuse. La teneur en agent dispersant peut avantageusement être comprise entre 0,001 % et 0,1 % en poids, plus avantageusement entre 0,001 % et 0,01 % en poids, par rapport au poids total de la suspension aqueuse.

L'agent dispersant est un agent tensio-actif hydrophile et non ionique, tel que par exemple le monooléate de sorbitane de polyoxyéthylène (polysorbate 80). De préférence la suspension contient 0,004% en poids de polysorbate 80, par rapport au poids total de la suspension.

Les étapes de dissolution (i), de dispersion (ii) et d'homogénéisation (iii) sont avantageusement réalisées à température ambiante et sous pression atmosphérique. Les conditions opératoires de dissolution, à l'étape i), puis de dispersion, étape ii), et enfin d'homogénéisation, étape iii), sont celles classiquement utilisées. En particulier, la dissolution, à l'étape i), est avantageusement réalisée sous mélange, à une vitesse comprise entre 100 et 400 tr/min, avantageusement à une vitesse d'environ 250 tr/min. Ensuite, le principe actif est introduit dans ledit réacteur (1) contenant l'agent dispersant dissous dans la composition aqueuse, l'ensemble (composition aqueuse, agent dispersant et principe actif introduit) étant soumis à une vive agitation, à une vitesse permettant d'obtenir un vortex, avantageusement environ 300 tr/min. L'actif est ajouté dans le réacteur. Les particules d'actif sont introduites dans le réacteur le plus souvent en moins de 5 minutes (en environ 1 min pour 250 à 400 g de particules d'actif). A la fin de l'introduction, la dispersion est homogénéisée (étape iii) sous agitation. Avantageusement, on utilise un système rotor/stator dont la taille des fentes est comprise entre 100 et 250 µm, avantageusement environ 200 µm. Le temps d'homogénéisation est suffisant pour obtenir une suspension homogène ; il est déterminé visuellement. Le temps d'homogénéisation est avantageusement d'au moins 15 minutes.

A la dispersion obtenue suite à cette étape d'homogénéisation est introduit, tel quel, l'agent de suspension. Lors de l'étape b) du procédé, l'agent de suspension est introduit sous sa forme commercialisée, le plus souvent une poudre. Il n'est pas nécessaire de le disperser préalablement, dans de l'eau par exemple.

L'agent de suspension peut être tout agent pharmaceutiquement acceptable capable de maintenir les particules du principe actif uniformément dispersées dans la formulation et de donner à la formulation les propriétés rhéologiques désirées. A titre d'exemple d'agent de suspension on peut citer la cellulose microcristalline, la carboxyméthylcellulose, le silicate d'aluminium et de magnésium, l'adragante, la bentonite, la méthylcellulose, les polyéthylène glycols, et leurs mélanges. Un agent de suspension préféré est un mélange de cellulose microcristalline et de carboxyméthylcellulose sodique, la cellulose microcristalline pouvant être présente en une quantité variant de 85 % à 95% en poids et la carboxyméthylcellulose sodique en une quantité variant de 5 % à 15% en poids, par rapport au poids total du mélange. L'agent de suspension peut être un excipient commercialisé sous la marque Avicel® CL 611, RC 591, RC 581, RC 501. La quantité d'agent de suspension varie avantageusement de 1 % à 5 % en poids, plus avantageusement de 1 % à 3,5 % en poids, par rapport au poids de la suspension aqueuse. La suspension comprend de préférence 2,0 % en poids d' Avicel® CL 611 (l'Avicel® CL 611 est un mélange de cellulose microcristalline - 85 % en poids par rapport au poids total du mélange- et de carmellose sodique - 15 % en poids par rapport au poids total du mélange-), par rapport au poids total de la suspension aqueuse. L'Avicel® CL 611 se caractérise par sa granulométrie (pas plus de 0,1 % en poids de particules passant au travers d'un tamis de 250 µm, pas plus de 50 % en poids de particules passant au travers d'un tamis de 65 µm), sa teneur en NaCMC (11,3 à 18,8 %), et sa viscosité (0,05 - 0,118 Pa.s).

L'agent de suspension, tel quel, est introduit dans la dispersion homogénéisée obtenue suite à l'étape b), qui est préalablement agitée à une vitesse supérieure ou égale à la vitesse d'homogénéisation. Cette vitesse est avantageusement comprise entre 300 et 600 tr/min, elle est plus avantageusement de 350 tr/min. L'agent de suspension est avantageusement ajouté rapidement, par exemple en quelques minutes, avantageusement en moins de 10 minutes (de 3 à 10 minutes pour 6 à 20 kg). Indiquer que l'agent de suspension est introduit tel quel signifie que cet agent de suspension n'a pas préalablement été mis en suspension dans une composition aqueuse. Au cours de l'introduction, réalisée à température ambiante et sous pression atmosphérique, l'ensemble (dispersion homogénéisée -composition aqueuse, agent dispersant et principe actif- et agent de suspension introduit) est soumis à une vive agitation, comprise entre 300 et 600 tr/min, avantageusement d'environ 350 tr/min.

Selon une variante avantageuse de l'invention, la suspension obtenue suite à cette étape b) est ensuite diluée par ajout d'eau. La quantité d'eau ajoutée correspond avantageusement à au moins 30 % en poids, plus avantageusement de 15 à 25 % en poids, de la teneur totale en eau dans la suspension obtenue à la fin du procédé selon l'invention. Ensuite, l'ensemble est mélangé (avantageusement au moins 20 minutes) et homogénéisé jusqu'à l'obtention d'une suspension homogène. La vitesse de mélange et d'homogénéisation est avantageusement comprise entre 200 et 400 tr/min, plus avantageusement elle est d'environ 250 tr/min. Avantageusement, pour homogénéiser, on utilise un système rotor/stator dont la taille des fentes est d'au moins 150 µm, plus avantageusement d'environ 200 µm. Le temps d'homogénéisation est déterminé visuellement : absence de résidu sur la grille. Généralement la solution diluée est mélangée au moins 15 minutes et homogénéisée au moins 5 minutes.

Le pH est avantageusement ajusté de manière à ce qu'il soit compris entre 4,5 et 5,5. Le plus souvent, le pH de la suspension obtenue précédemment est supérieur à 5,5, on ajoute donc en quantité nécessaire et suffisante un acide, tel que l'acide chlorhydrique, avantageusement dilué dans de l'eau. Si le pH est inférieur à 4,5, il est ajusté par ajout d'une solution d'hydroxyde de sodium.

Ensuite, la masse finale de suspension peut être ajustée par ajout, si nécessaire, d'eau. Enfin, la suspension est conditionnée. Ces opérations sont avantageusement effectuées sous agitation, par exemple à une vitesse comprise entre 100 et 400 tr/min.

Toutes ces étapes sont avantageusement réalisées à température ambiante et sous pression atmosphérique.

Par la suite, on décrit les conditions opératoires d'une variante préférée du procédé, dans laquelle on utilise un réacteur de 600 litres (pour une fabrication d'environ 450 kg de suspension), équipé d'un mélangeur à hélice et d'un agitateur par cisaillement (tel qu'un agitateur Ultra Turrax commercialisé par SYLVERSON ; homogénéisateur).

Dans le réacteur de 600 litres, on introduit 380 à 460 litres d'eau puis 0,006 à 0,030 kg d'agent dispersant. L'étape i) de dissolution est réalisée sous agitation à l'aide du mélangeur à hélice. La vitesse de mélange varie avantageusement de 100 à 400 tr/min (tours par minute) et le temps de mélange est suffisant pour obtenir une solution limpide, il est avantageusement d'au moins 10 minutes. Ensuite, l'agitateur par cisaillement est déclenché et le principe actif est introduit en quelques minutes dans ledit réacteur (1), avantageusement en moins d'une minute. La vitesse de l'agitateur est telle qu'il se forme un vortex sans formation de mousse, elle est avantageusement de 300 tr/min. A la fin de l'introduction, l'agitation est maintenue, avantageusement à la même vitesse, pour assurer l'homogénéisation de la dispersion. La taille des maille du mobile d'homogénéisation est comprise entre 100 et 250 µm. Le temps d'homogénéisation est avantageusement d'au moins 15 minutes.

Dans le réacteur (1) précédent, maintenu sous agitation avec l'agitateur Ultra Turrax, avantageusement à une vitesse plus élevée, comprise entre 300 et 600 tr/min, on introduit 6 à 20 kg d'agent de suspension. Avantageusement, l'agent de suspension est introduit en 3 à 10 minutes.

Puis, on introduit dans ce même réacteur, maintenu sous agitation avec l'agitateur Ultra Turrax, avantageusement à la même vitesse ou à une vitesse réduite, comprise entre 200 et 400 tr/min, 80 à 140 litres d'eau. L'ensemble est mélangé (pendant au moins 20 minutes) puis homogénéisé (taille des mailles d'au moins 150 µm) jusqu'à l'obtention d'une suspension homogène.

On mesure ensuite le pH de la suspension. Si celui-ci est en dehors des spécifications (pH compris entre 4,5 et 5,5), on ajuste le pH. Le plus souvent, le pH de la suspension est trop basique, on ajoute donc la quantité nécessaire et suffisante d'acide chlorhydrique dilué dans l'eau.

Ensuite, la masse finale est, le cas échéant, ajustée par ajout d'eau, sous agitation d'environ 200 tr/min.

Enfin, la suspension est conditionnée. On peut dans un premier temps procéder à la répartition de la suspension en flacon pulvérisateur au volume théorique de 15 ml (conditionnement primaire) puis conditionner ces flacons dans des étuis en carton en insérant une notice (conditionnement secondaire).

Pour protéger la formulation contre la contamination et la croissance microbienne, on peut en outre introduire des agents antimicrobiens pharmaceutiquement acceptables. A titre d'exemple, on peut notamment citer les composés d'ammonium quaternaire (le chlorure de benzalkonium, le chlorure de benzethonium, le cetrimide, et le chlorure de cetylpyridinium), les composés du mercure (nitrate phénylmercurique, acétate phénylmercurique, thimérosal), les agents alcooliques (chlorobutanol, alcool phényléthylique, alcool benzylique), les esters antibactériens (esters d'acide parahydroxybenzoïque) et d'autres agents antimicrobiens tels que la chlorhexidine, le chlorocrésol, et la polymyxine. La teneur en agent antimicrobien peut varier d'environ 0,001% à environ 1 %, plus avantageusement d'environ 0,001% à environ 0,1 % en poids du poids total de la suspension aqueuse.

Selon une variante avantageuse, lorsque l'agent antimicrobien est un composé d'ammonium quaternaire on lui associe un agent chélatant. Ceci permet de renforcer la stabilité de la suspension et de s'affranchir de la présence d'un agent antioxydant. De manière préférée, la suspension contient le chlorure de benzalkonium et le tétraacétate disodique d'éthylènediamine (EDTA). La suspension comprend avantageusement 0,001 % à environ 0,03 % en poids du composé d'ammonium quaternaire et environ 0,01 % à environ 0,1 % en poids de l'agent chélatant, par rapport au poids total de la suspension aqueuse. La suspension comprend de préférence 0,015 % en poids de chlorure de benzalkonium et 0,050 % en poids d'EDTA, par rapport au poids total de la suspension aqueuse.

La suspension inclut avantageusement en outre un agent iso-osmotique destiné à prévenir l'irritation des muqueuses nasales par la suspension. Cet excipient pourra être choisi dans le groupe constitué par le glucose anhydre, le dextrose anhydre, le chlorure de sodium, le chlorure de calcium, et leurs mélanges, avantageusement le glucose anhydre. La teneur en cet excipient dans la suspension pourra aller jusqu'à environ 8 % en poids par rapport au poids de la suspension aqueuse. La suspension comprend de préférence 5 % en poids de glucose anhydre, par rapport au poids total de la suspension aqueuse.

La suspension peut également comprendre d'autres excipients tels qu'un agent antioxydant. A titre d'exemple, on peut notamment citer l'acide ascorbique, l'ascorbate de sodium, le bisulfite de sodium, le thiosulfate de sodium, la quinoline 8-hydroxy, le cystéine de N-acétyle, l'alpha tocophérol, et leurs mélanges.

Les excipients, outre l'agent dispersant, pouvant être utilisés sont choisis dans le groupe constitué par un agent anti-microbien, un agent chélatant, un agent iso-osmotique, un agent anti-oxydant et leurs mélanges.

Tous ces excipients sont introduits dès le début du procédé, lors de l'étape a), en particulier lors de l'étape i). Leur introduction ne nécessite pas de modification du procédé selon l'invention décrit précédemment. L'étape i), du procédé selon l'invention, comprend donc, de manière avantageuse, la dissolution dans l'eau de l'agent dispersant et de tous les autres excipients. En particulier, dans le procédé selon l'invention l'étape a), en particulier l'étape i), comprend en outre la dissolution dans l'eau d'un ou plusieurs excipients choisis dans le groupe constitué par un agent anti-microbien, un agent chélatant, un agent iso-osmotique, un agent anti-oxydant et leurs mélanges.

La suspension obtenue par le procédé selon l'invention présente un comportement rhéofluidifiant. Les essais réalisés sur la suspension de l'exemple 2 montrent un comportement rhéofluidifiant (i.e. la viscosité diminue avec la vitesse de déformation) avec une petite boucle d'hystérésis suggérant des propriétés thixotropes faibles.

Lorsqu'une suspension est soumise à une contrainte (ou gradient de vitesse), on dit que cette suspension a un comportement rhéofluidifiant lorsque sa viscosité varie avec une variation de la contrainte appliquée. Une suspension a un comportement dit thixotrope si sa viscosité, à vitesse de cisaillement constante, diminue au cours du temps à condition que ce phénomène soit réversible.

La suspension selon l'invention présente une viscosité élevée au repos, lui permettant d'adhérer aux parois nasales. La viscosité au repos est avantageusement supérieure à 120 mPa.s. Sous agitation (lorsque le récipient la contenant est remué), sa viscosité diminue ce qui permet sa pulvérisation. La viscosité sous agitation est avantageusement inférieure à 90 mPa.s.

La suspension obtenue par le procédé selon l'invention peut être délivrée par tout dispositif pompe/diffuseur approprié, tels que ceux commercialisés par la société APTAR PHARMA (et décrites dans le brevet européen n° 0 938 345). De tels dispositifs contiennent un récipient contenant ladite suspension aqueuse et une pompe de pré-composition associée au récipient capable de projeter par pulvérisation une dose complète de la suspension (avantageusement 100 mg) dans la narine d'une personne.

La pompe est associée à un dispositif de diffusion avantageusement de forme conique. Le dispositif doit permettre d'obtenir un spray pour lequel les spécifications suivantes sont respectées :
- taille des gouttelettes : à une distance de 20 mm, un Dv(10) compris entre 15 et 25 µm, un Dv(50) compris entre 50 et 65 µm, un Dv(90) compris entre 110 et 130 µm, et à une distance de 50 mm, un Dv(10) compris entre 15 et 25 µm, un Dv(50) compris entre 40 et 55 µm, un Dv(90) compris entre 85 et 115 µm ;
- l'angle du cône de dispersion, en anglais « plume geometry », est compris entre 40° et 55° ;
- une forme de spray, en anglais « spray pattern », telle que le ratio X/Y soit compris entre 1 et 1,5 ; où X représente la longueur maximale du grand axe et Y représente la longueur maximale du petit axe, X et Y étant les axes de l'ellipse formée par le spray.

Les exemples qui suivent illustrent l'invention mais ne sont pas limitatifs.

### Exemple 1 : Procédé de fabrication - exemple de l'acétonide de triamcinolone

Le procédé de fabrication consiste à la préparation d'une solution aqueuse contenant l'édétate disodique, le polysorbate 80, le chlorure de benzalkonium et le glucose anhydre. L'acétonide de triamcinolone est dispersée dans cette solution. La suspension ainsi obtenue est épaissie par ajout d'Avicel® CL 611. Le procédé de fabrication est poursuivi par l'ajustement du pH et de la masse finale.

La fabrication est réalisée selon les bonnes pratiques de fabrication. Le procédé de fabrication est détaillé ci-après. Les quantités indiquées sont données à titre d'exemple pour une fabrication de 600 kg. On vérifie la propreté du matériel et de la zone de travail. On pèse ensuite les constituants selon les valeurs désirées.

Dans la cuve de fabrication munie d'un mélangeur à hélice, on introduit 450,0 l d'eau purifiée. On ajoute ensuite successivement sous agitation (en laissant dissoudre totalement après chaque ajout) :
- Edétate disodique : 0,3000 kg
- Polysorbate 80 : 0,0240 kg
- Chlorure de Benzalkonium : 0,1800 kg
- Glucose anhydre : 30,0000 kg

On mélange 15 min. On obtient une solution limpide.

Dans la cuve de fabrication précédente, on déclenche l'agitateur SYLVERSON (300 tr/min) et on ajoute 0,3300 kg d'acétonide de triamcinolone micronisé. On maintient l'agitation jusqu'à l'obtention d'une suspension homogène (15 min minimum). La taille des fentes est de 200 µm. On vérifie visuellement que la suspension ne contienne plus de résidu.

Dans la cuve de fabrication précédente, maintenue sous agitation avec l'agitateur SYLVERSON (300 tr/min), on augmente la vitesse d'agitation (350 tr/min) puis on introduit rapidement (5 min) 12,000 kg d'Avivel® CL 611. A la fin de l'ajout, on réduit la vitesse d'agitation (250 tr/min) puis on introduit 100,0 kg d'eau purifiée. On mélange jusqu'à l'obtention d'une suspension homogène (environ 20 min). La taille des fentes est de 200 µm. On vérifie visuellement l'absence de résidu.

On mesure le pH de la suspension obtenue à l'étape précédente, celui-ci doit être compris entre 4,5 et 5,5. On ajuste le pH à la spécification requise en ajoutant lentement une solution d'acide chlorhydrique et/ou une solution d'hydroxyde de sodium. On maintient l'agitation jusqu'à l'obtention d'une suspension homogène (10 min).

On ajoute la quantité d'eau purifiée pour atteindre un poids de 600,000 kg. On maintient l'agitation (à environ 200 tr/min) jusqu'au conditionnement.

On procède ensuite à la répartition de la suspension en flacon pulvérisateur au remplissage théorique (16,5 g de suspension par flacon). On conditionne enfin les flacons en étuis carton en insérant une notice.

### Exemple 2 : Suspension aqueuse d'acétonide de triamcinolone obtenue par le procédé selon l'invention

Par le procédé décrit dans l'exemple 1, on obtient un flacon de suspension aqueuse dont la composition est donnée dans le tableau 1 :

**Tableau 1**

| **Nom des composants** | **Quantité pour 100 g** |
|---|---|
| Triamcinolone (Acétonide de) | 0,055 |
| Edétate sodique | 0,050 |
| Glucose anhydre | 5,000 |
| Cellulose microcristalline et Carmellose sodique : Avicel® CL 611* | 2,000 |
| Polysorbate 80 | 0,004 |
| Chlorure de benzalkonium, solution à 50% (Chlorure de benzalkonium) | 0,030 (0,015) |
| Acide chlorhydrique dilué à 10% | qsp pH 5,0 ± 0,5 |
| Eau purifiée | qsp 100,000 g (environ 93,00 g) |

| | |
|---|---|
| * Avicel® CL 611 est un mélange de cellulose microcristalline (85% en poids) et de carmellose sodique (15% en poids). | |

Une étude rhéologique des propriétés d'écoulement de cette suspension A et du produit de référence (Nasacort®, objet du brevet EP 0 938 345) a été conduite.

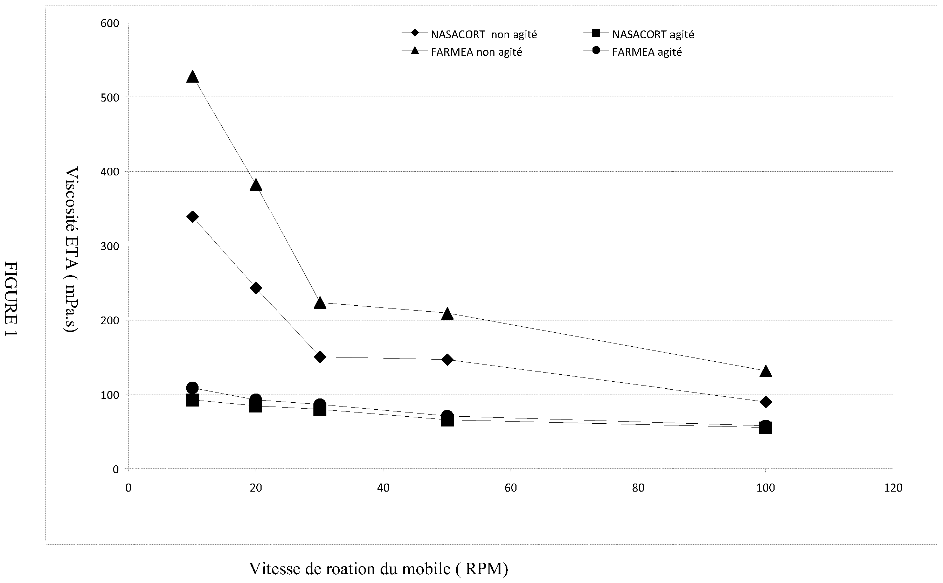

Les mesures de viscosité ont été réalisées sur un viscosimètre Brookfield à 30 tours par minute pendant 30 secondes sur les 2 produits avant et après agitation. Les résultats sont rapportés sur le tableau 2 et la figure 1.

**Tableau 2**

| **ECHANTILLON** | | **Viscosité Brookfield (mPa.s)** | **Ecart Type** |
|---|---|---|---|
| Nasacort® | Non agité | 150,4 | 1,6 |
| | Agité | 80,0 | 1,6 |
| Suspension A | Non agité | 224,0 | 2,1 |
| | Agité | 86,4 | 1,6 |

La figure 1 représente l'évolution de la viscosité ETA (mPa.s) en fonction de la vitesse de rotation du mobile (RPM) pour : le Nasacort® non agité (trait plein), le Nasacort® agité (trait discontinu), la suspension A non agitée (pointillés), la suspension A agitée (alternance pointillés- tirets).

La suspension A présente des caractéristiques de thixotropie comparables à celles du produit de référence, Nasacort®.

### Exemple 3 : Caractéristiques de la pompe et du diffuseur

La pompe utilisée dans cet exemple est une pompe fabriquée par APTAR PHARMA, sous la référence VP7/100S 18/415 avec un diffuseur 32 NA/B/R + B63 et un clip.

Les caractéristiques étudiées et compares à celles du produit de référence (Nasacort®) sont:
- angle du cône de dispersion (plume geometry) ;
- forme de spray (spray pattern) ;
- taille des gouttelettes (droplet size distribution).

Ces études ont été réalisées en suivant les directives de la qualité pharmaceutique des produits destinés à une administration par inhalation et par voie nasale (EMEA/CHMP/QWP/49313/2005)

### ▪ angle du cône de dispersion (Plume geometry)

L'angle du cône de dispersion a été étudié sur la suspension aqueuse obtenu par le procédé décrit dans l'exemple 1 (composition donnée dans l'exemple 2) conditionnée avec 3 lots de pompe différents. Une comparaison au produit de référence a été réalisée. Les analyses ont été réalisées en utilisant un système automatique de fourniture de la dose (Proveris Type NSX). Les résultats sont acquis par un logiciel adapté. Les paramètres sont détaillés dans le tableau 3 suivant :

**Tableau 3: paramètres**

| Paramètres de libération | Paramètres d'acquisition |
|---|---|
| Vitesse de libération: 70 mm/s | Spray: vertical |
| Accélération: 5000 mm/s² | Fréquence d'acquisition: 100 images / seconde |
| Temps de rétention: 0,3 s | Seuil de bruit: 1 |

Les résultats sont reportés dans les tableaux 4 et 5 suivants.

**Tableaux 4 et 5: résultats pour la suspension A ou pour la référence Nasacort®**

| **Suspension A** | **Test** | **Angle (°)** |
|---|---|---|
| A-1 | 1 | 50 |
| | 2 | 46 |
| | 3 | 50 |
| A-2 | 1 | 42 |
| | 2 | 41 |
| | 3 | 41 |
| A-3 | 1 | 46 |
| | 2 | 46 |
| | 3 | 47 |
| Moyenne | | 45 |
| Ecart-type | | 3 |

| **Nasacort®** | **Test** | **Angle(°)** |
|---|---|---|
| N-1 | 1 | 50 |
| | 2 | 49 |
| | 3 | 50 |
| N-2 | 1 | 55 |
| | 2 | 55 |
| | 3 | 54 |
| N-3 | 1 | 51 |
| | 2 | 50 |
| | 3 | 52 |
| Moyenne | | 52 |
| Ecart-type | | 2 |

L'angle de cône de dispersion pour la suspension A est comparable à celui obtenu pour le produit de référence.

### ▪ forme de spray (Spray pattern)

La forme du spray a été étudiée sur la suspension aqueuse obtenu par le procédé décrit dans l'exemple 1 (composition donnée dans l'exemple 2) conditionnée avec 3 lots de pompe différents. Une comparaison au produit de référence a été réalisée. Les analyses ont été réalisées en utilisant un système automatique de fourniture de la dose (Proveris Type NSX). Les résultats sont acquis par un logiciel adapté. Les paramètres sont détaillés dans le tableau 6 suivant :

**Tableau 6: paramètres**

| Paramètres de libération | Paramètres d'acquisition |
|---|---|
| Vitesse de libération: 70 mm/s | Distance Laser - tête de la pompe : 5 cm et 3 cm |
| Accélération: 5000 mm/s² | Spray: vertical |
| Temps de rétention: 0,3 s | Fréquence d'acquisition: 100 images / seconde |
| | Seuil de bruit: 1 |

Résultats à 3 cm:

**Tableaux 7 et 8: résultats à 3 cm pour la suspension ou pour la référence**

| **A** | **Diam. Max (X mm)** | **Diam. Min (Y mm)** | **X/Y** |
|---|---|---|---|
| A-1 | 28 | 25 | 1,2 |
| | 30 | 25 | 1,2 |
| | 29 | 25 | 1,1 |
| A-2 | 27 | 23 | 1,2 |
| | 26 | 23 | 1,1 |
| | 26 | 23 | 1,1 |
| A-3 | 28 | 24 | 1,2 |
| | 27 | 24 | 1,2 |
| | 28 | 24 | 1,2 |
| Moyenne | 28 | 24 | 1,2 |
| Ecart-type | 1 | 1 | 0,0 |

| **Nasacort^{®}** | **Diam. Max (X mm)** | **Diam. Min (Y mm)** | **X/Y** |
|---|---|---|---|
| N-1 | 29 | 23 | 1,2 |
| | 29 | 23 | 1,3 |
| | 28 | 23 | 1,2 |
| N-2 | 34 | 22 | 1,5 |
| | 32 | 21 | 1,5 |
| | 33 | 21 | 1,6 |
| N-3 | 31 | 25 | 1,2 |
| | 31 | 25 | 1,2 |
| | 31 | 25 | 1,3 |
| Moyenne | 31 | 23 | 1,3 |
| Ecart-type | 2 | 2 | 0,1 |

Résultats à 5 cm:

**Tableaux 9 et 10: résultats à 5 cm pour la suspension ou pour la référence**

| **A** | **Diam. Max (X mm)** | **Diam. Min (Y mm)** | **X/Y** |
|---|---|---|---|
| A-1 | 44 | 39 | 1,1 |
| | 43 | 39 | 1,1 |
| | 46 | 39 | 1,2 |
| A-2 | 40 | 36 | 1,1 |
| | 40 | 34 | 1,2 |
| | 39 | 34 | 1,1 |
| A-3 | 44 | 36 | 1,2 |
| | 43 | 37 | 1,2 |
| | 42 | 38 | 1,1 |
| Moyenne | 42 | 37 | 1,2 |
| Ecart-type | 2 | 2 | 0,0 |

| **Nasacort®** | **Diam. Max (X mm)** | **Diam. Min (Y mm)** | **X/Y** |
|---|---|---|---|
| N-1 | 44 | 34 | 1,3 |
| | 42 | 35 | 1,2 |
| | 41 | 32 | 1,3 |
| N-2 | 44 | 29 | 1,5 |
| | 44 | 90 | 1,4 |
| | 44 | 91 | 1,4 |
| N-3 | 45 | 38 | 1,2 |
| | 45 | 37 | 1,2 |
| | 45 | 37 | 1,2 |
| Moyenne | 44 | 34 | 1,3 |
| Ecart-type | 2 | 3 | 0,1 |

Les valeurs moyennes X/Y pour la suspension A, obtenue par le procédé selon l'invention, sont comparables à celles obtenues pour le produit de référence, pour les distances de 3 cm et 5 cm.

### ▪ taille des gouttelettes (Droplet size distribution)

La taille des gouttelettes a été évaluée à une distance de 2 cm et de 5 cm pour 3 lots différents de flacon de suspension aqueuse obtenu par le procédé décrit dans l'exemple 1 (composition donnée dans l'exemple 2). Les résultats sont donnés dans le tableau 11 suivant.

**Tableau 11: résultats pour la suspension A obtenue par le procédé selon l'invention**

| **Suspension A** | | **Distance 20 mm** | | | **Distance 50 mm** | | |
|---|---|---|---|---|---|---|---|
| Lot | essai | Dv(10) µm | Dv(50) µm | Dv(90) µm | Dv(10) µm | Dv(50) µm | Dv(90) µm |
| A-1 | 1 | 21,18 | 60,03 | 122,60 | 20,95 | 45,65 | 98,14 |
| | 2 | 21,92 | 58,27 | 119,40 | 21,91 | 45,15 | 92,27 |
| | 3 | 20,17 | 54,35 | 111,80 | 21,77 | 44,29 | 88,12 |
| A-2 | 1 | 21,43 | 59,66 | 120,70 | 22,37 | 46,05 | 96,55 |
| | 2 | 23,63 | 64,54 | 128,20 | 21,73 | 48,51 | 105,00 |
| | 3 | 20,07 | 56,09 | 117,30 | 21,22 | 43,73 | 89,55 |
| A-3 | 1 | 19,76 | 56,26 | 117,50 | 21,88 | 44,16 | 91,65 |
| | 2 | 20,31 | 58,39 | 120,40 | 20,37 | 43,99 | 94,79 |
| | 3 | 20,20 | 56,07 | 114,70 | 20,59 | 43,53 | 91,00 |

Dv(10): 10% des particules ont un diamètre inférieur à Dv en µm.
Dv(50): 50% des particules ont un diamètre inférieur à Dv en µm.
Dv(90): 90% des particules ont un diamètre inférieur à Dv en µm.

Les résultats obtenus sont comparés à ceux obtenus pour le produit de référence (Nasacort®), donnés dans le tableau 12 suivant.

**Tableau 12 : résultats pour le produit de référence Nasacort®**

| **Distance 20 mm** | | | | | **Distance 50 mm** | | |
|---|---|---|---|---|---|---|---|
| **Nasacort®** | Trial | Dv(10) µm | Dv(50) µm | Dv(90) µm | Dv(10) µm | Dv(50) µm | Dv(90) µm |
| | 1 | 19,04 | 56,93 | 119,00 | 24,31 23,51 23,07 | 52,32 | 112,8 |
| | 2 | 22,81 | 64,35 | 129,20 | | 50,21 | 104,7 |
| | 3 | 20,69 | 55,61 | 114,50 | | 45,34 | 90,78 |

Les résultats obtenus pour la suspension obtenue par le procédé selon l'invention et le produit de référence sont similaires.

### ▪ Conclusion sur l'étude de la pompe/diffuseur

La pompe et le diffuseur utilisés permettent d'obtenir avec la suspension A, obtenue par le procédé selon l'invention, les mêmes caractéristiques de spray que celles obtenues avec le produit de référence Nasacort®, à savoir angle du cône de dispersion (plume geometry), forme de spray (spray pattern) et taille des gouttelettes (droplet size distribution).

## Revendications

1. Procédé industriel de préparation d'une suspension aqueuse d'agent anti-inflammatoire stéroïdien comprenant un agent dispersant, un agent de suspension, et un ou plusieurs excipients choisis dans le groupe constitué par des agents antimicrobiens, des agents chélatants, des agents iso-osmotiques, des agents antioxydants, et leurs mélanges, **caractérisé en ce qu'**il comprend les étapes successives suivantes :
a) préparation dans un réacteur (1) d'une dispersion comprenant
- de l'eau,
- tous lesdits un ou plusieurs excipients;
- l'agent dispersant qui est un agent tensio-actif hydrophile et non ionique, et
- l'agent anti-inflammatoire stéroïdien sous forme de particules ;
b) ajout, à la dispersion obtenue suite à l'étape a), de l'agent de suspension tel quel dans ce même réacteur (1) ;
c) le cas échéant dilution, par ajout d'eau dans ce même réacteur (1), et/ou ajustement du pH de ladite suspension obtenue suite à l'étape b), et récupération de la suspension aqueuse.

2. Procédé selon la revendication 1, **caractérisé en ce que** l'étape a) comprend trois étapes successives distinctes :
i) dissolution dans l'eau de l'agent dispersant et de tous lesdits un ou plusieurs excipients ; puis
ii) ajout de l'agent anti-inflammatoire stéroïdien à la solution obtenue suite à l'étape i) et formation de la dispersion ; et
iii) homogénéisation de la dispersion formée à l'étape ii).

3. Procédé selon les revendications 1 ou 2, **caractérisé en ce que** l'agent anti-inflammatoire stéroïdien est choisi dans le groupe constitué par l'acétonide de triamcinolone et le mométasone furoate monohydraté, de préférence l'acétonide de triamcinolone.

4. Procédé selon l'une quelconque des revendications précédentes, **caractérisé en ce que** la teneur en agent anti-inflammatoire stéroïdien dans la suspension est comprise entre 0,001 % et 2 % poids, de préférence entre 0,01 % et 0,2 % en poids, plus préférentiellement de 0,055% en poids, par rapport au poids total de la suspension aqueuse.

5. Procédé selon l'une quelconque des revendications précédentes, **caractérisé en ce que** la teneur en agent dispersant est comprise entre 0,001 % et 0,1 % en poids, avantageusement entre 0,001 % et 0,01 % en poids, par rapport au poids total de la suspension aqueuse.

6. Procédé selon l'une quelconque des revendications précédentes, **caractérisé en ce que** l'agent dispersant est le polysorbate 80.

7. Procédé selon l'une quelconque des revendications précédentes, **caractérisé en ce que** l'agent de suspension est un mélange de cellulose microcristalline et de carboxyméthylcellulose sodique, la cellulose microcristalline étant présente en une quantité variant de 85 à 95 % en poids et la carboxyméthylcellulose sodique en une quantité variant de 5 à 15 % en poids, par rapport au poids total du mélange.

8. Procédé selon l'une quelconque des revendications précédentes, **caractérisé en ce que** la quantité d'agent de suspension varie de 1 à 5 % en poids par rapport au poids total de la suspension aqueuse.

9. Procédé selon l'une quelconque des revendications 1 à 8, **caractérisé en ce que** l'agent anti-microbien est le chlorure de benzalkonium.

10. Procédé selon l'une quelconque des revendications 1 à 8, **caractérisé en ce que** l'agent chélatant est l'EDTA

11. Procédé selon l'une quelconque des revendications 1 à 8, **caractérisé en ce que** l'agent iso-osmotique est le glucose anhydre.

## Patentansprüche

1. Industrielles Zubereitungsverfahren einer wässrigen Suspension aus einem antiinflammatorischem, steroidalen Wirkstoff, umfassend einen Dispergierer, ein Schwebemittel und einen oder mehrere Hilfsstoff(e), die aus der Gruppe ausgewählt sind, bestehend aus antimikrobiellen Wirkstoffen, chelatbildenden Wirkstoffen, iso-osmotischen Wirkstoffen, Antioxidantien und deren Mischungen, **dadurch gekennzeichnet, dass** es die folgenden sukzessiven Schritte umfasst:
a) Zubereitung einer Dispergierung in einem Reaktionsgefäß (1), umfassend:
- Wasser,
- alle die genannten einzigen oder mehreren Hilfsstoff(e) ;
- den Dispergierer, der ein oberflächenaktiver, hydrophiler und nicht ionischer Wirkstoff ist, und
- der anti-inflammatorische, steroidale Wirkstoff in Partikelform;
b) Hinzufügen des unveränderten Schwebemittels in dasselbe Reaktionsgefäß (1) zu der in Schritt a) erhaltenen Dispergierung;
c) ggf. Verdünnung durch Hinzufügen von Wasser in dasselbe Reaktionsgefäß (1) und / oder Anpassen des ph-Wertes der genannten, im Anschluss an Schritt b) erhaltene Suspension und Auffangen der wässrigen Suspension.

2. Verfahren gemäß Anspruch 1, **dadurch gekennzeichnet, dass** der Schritt a) drei unterschiedliche, sukzessive Schritte umfasst:
i) Auflösung des Dispergierers und aller der genannten einzigen oder mehreren Hilfsstoff(e) in Wasser; dann
ii) Hinzufügen des anti-inflammatorischen, steroidalen Wirkstoffs zu der im Anschluss an den Schritt i) erhaltenen Lösung und Bildung der Dispergierung; und
iii) Homogenisierung der in Schritt ii) gebildeten Dispergierung.

3. Verfahren gemäß Anspruch 1 oder 2, **dadurch gekennzeichnet, dass** der anti-inflammatorische, steroidale Wirkstoff aus der Gruppe ausgewählt ist, bestehend aus Triamcinolonacetonid und kristallwasserhaltigem Mometasenonfuroat, bevorzugt Triamcinolonacetonid.

4. Verfahren gemäß irgendeinem der voranstehenden Ansprüche, **dadurch gekennzeichnet, dass** der Gehalt an antiinflammatorischem, steroidalen Wirkstoff in der Suspension zwischen 0,001 Gew.-% und 2 Gew.-%, bevorzugt zwischen 0,01 Gew.-% und 0,2 Gew.-%, weiter bevorzugt 0,055 Gew.-% im Verhältnis zum Gesamtgewicht der wässerigen Suspension inbegriffen ist.

5. Verfahren gemäß irgendeinem der voranstehenden Ansprüche, **dadurch gekennzeichnet, dass** der Gehalt an Dispergierer zwischen 0,001 Gew.-% und 0,1 Gew.-%, vorteilhaft zwischen 0,001 Gew.-% und 0,01 Gew.-% im Verhältnis zum Gesamtgewicht der wässerigen Suspension inbegriffen ist.

6. Verfahren gemäß irgendeinem der voranstehenden Ansprüche, **dadurch gekennzeichnet, dass** der Dispergierer Polysorbat 80 ist.

7. Verfahren gemäß irgendeinem der voranstehenden Ansprüche, **dadurch gekennzeichnet, dass** das Schwebemittel eine Mischung aus mikrokristalliner Zellulose und Natrium-Carboxymethylzellulose ist, wobei die mikrokristalline Zellulose in einer zwischen 85 und 95 Gew.-% variierenden Menge und die Natrium-Carboxymethylzellulose in einer zwischen 5 und 15 Gew.-% variierenden Menge im Verhältnis zum Gesamtgewicht der Mischung vorhanden ist.

8. Verfahren gemäß irgendeinem der voranstehenden Ansprüche, **dadurch gekennzeichnet, dass** die Menge an Schwebemittel von 1 bis 5 Gew.-% im Verhältnis zum Gesamtgewicht der wässerigen Suspension variiert.

9. Verfahren gemäß irgendeinem der Ansprüche 1 bis 8, **dadurch gekennzeichnet, dass** der antimikrobielle Wirkstoff Benzalkoniumchlorid ist.

10. Verfahren gemäß irgendeinem der Ansprüche 1 bis 8, **dadurch gekennzeichnet, dass** der chelatbildende Wirkstoff EDTA ist.

11. Verfahren gemäß irgendeinem der Ansprüche 1 bis 8, **dadurch gekennzeichnet, dass** der iso-osmotische Wirkstoff wasserfreie Glukose ist.

## Claims

1. An industrial process for preparing an aqueous suspension of steroidal anti-inflammatory agent comprising a dispersing agent, a suspension agent, and one or more excipients chosen from the group consisting of antimicrobial agents, chelating agents, iso-osmotic agents, antioxidant agents, and mixtures thereof, **characterised in that** it comprises the following successive steps of:
a) preparing in a reactor (1) a dispersion comprising
- water,
- all said one or more excipients;
- the dispersing agent which is a hydrophilic nonionic surfactant, and
- the steroidal anti-inflammatory agent as particles;
b) adding to the dispersion obtained following step a), the suspension agent as such in this same reactor (1);
c) optionally, diluting, by adding water in this same reactor (1), and/or adjusting the pH of said suspension obtained following step b), and recovering the aqueous suspension.

2. The process according to claim 1, **characterised in that** step a) comprises three distinct successive steps of:
i) dissolving in water the dispersing agent and all said one or more excipients; then
ii) adding the steroidal anti-inflammatory agent to the solution obtained following step i) and forming the dispersion; and
iii) homogenising the dispersion formed in step ii).

3. The process according to claims 1 or 2, **characterised in that** the steroidal anti-inflammatory agent is chosen from the group consisting of triamcinolone acetonide and mometasone furoate monohydrate, preferably triamcinolone acetonide.

4. The process according to any of the preceding claims, **characterised in that** the content of steroidal anti-inflammatory agent in the suspension is between 0.001 % and 2 %-wt, preferably between 0.01 % and 0.2 %-wt, more preferentially 0.055 %-wt, relative to the total weight of the aqueous suspension.

5. The process according to any of the preceding claims, **characterised in that** the content of dispersing agent is between 0.001 % and 0.1 %-wt, advantageously between 0.001 % and 0.01 %-wt, relative to the total weight of the aqueous suspension.

6. The process according to any of the preceding claims, **characterised in that** the dispersing agent is polysorbate 80.

7. The process according to any of the preceding claims, **characterised in that** the suspension agent is a mixture of microcrystalline cellulose and sodium carboxymethylcellulose, microcrystalline cellulose being present in an amount ranging from 85 to 95 %-wt and the sodium carboxymethylcellulose in an amount ranging from 5 to 15 %-wt, relative to the total weight of the mixture.

8. The process according to any of the preceding claims, **characterised in that** the amount of suspension agent ranges from 1 to 5 %wt relative to the total weight of the aqueous suspension.

9. The process according to any of claims 1 to 8, **characterised in that** the antimicrobial agent is benzalkonium chloride.

10. The process according to any of claims 1 to 8, **characterised in that** the chelating agent is EDTA.

11. The process according to any of claims 1 to 8, **characterised in that** the iso-osmotic agent is anhydrous glucose.
